(19) Europäisches Patentamt
European Patent Office
Office européen des brevets

(11) Veröffentlichungsnummer : **0 161 543**
**B1**

(12) # EUROPÄISCHE PATENTSCHRIFT

(45) Veröffentlichungstag der Patentschrift : **02.03.88**

(21) Anmeldenummer : **85104900.7**

(22) Anmeldetag : **23.04.85**

(51) Int. Cl.[4] : **C 07 C 69/74**, C 07 C 69/65, C 07 C 121/75, A 01 N 53/00, A 01 N 37/10, A 01 N 37/34

(54) **Neue Halogenvinylbenzylester, deren Herstellung und Verwendung zur Schädlingsbekämpfung.**

(30) Priorität : **14.05.84 DE 3417791**

(43) Veröffentlichungstag der Anmeldung : **21.11.85 Patentblatt 85/47**

(45) Bekanntmachung des Hinweises auf die Patenterteilung : **02.03.88 Patentblatt 88/09**

(84) Benannte Vertragsstaaten : **BE CH DE FR GB IT LI NL**

(56) Entgegenhaltungen :
**DE-A- 3 225 129**
**FR-A- 2 293 416**
**FR-A- 2 319 622**
**GB-A- 1 078 511**
**GB-A- 1 518 509**
**GB-A- 2 121 039**
**GB-A- 2 122 616**

(73) Patentinhaber : **BASF Aktiengesellschaft**
**Carl-Bosch-Strasse 38**
**D-6700 Ludwigshafen (DE)**

(72) Erfinder : **Schwarz, Gerd-Ulrich, Dr.**
**Keltenstrasse 13**
**D-6707 Schifferstadt (DE)**
Erfinder : **Theobald, Hans, Dr.**
**Queichstrasse 6**
**D-6703 Limburgerhof (DE)**
Erfinder : **Adolphi, Heinrich, Dr.**
**Kalmitweg 11**
**D-6703 Limburgerhof (DE)**

**EP 0 161 543 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es ist bekannt, daß 2,2-Dimethyl-3-(2′,2′-dimethylvinyl)-cyclopropancarbonsäureester, die als Alkohol einen substituierten Vinylbenzylrest enthalten, insektizid wirksam sind (GB 2 065 475, BE 0 738 112).

Die Kombination solcher Benzylalkohole mit speziellen Carbonsäuren führt zu Schädlingsbekämpfungsmitteln mit besonders guter Wirkung.

Es wurde gefunden, daß neue Ester der Formel I

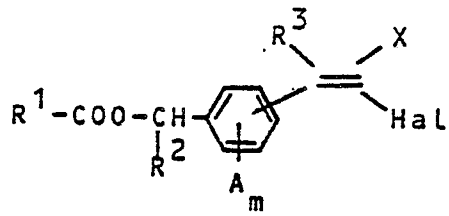

(I)

worin

$R^1$

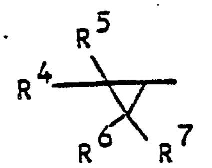
,
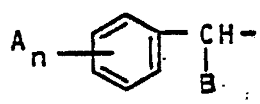
,

$R^2$ H, CN, nieder-Alkyl, -Alkenyl, -Alkinyl, -Halogenalkyl, -Halogenalkenyl,

$R^3$ H, Halogen, nieder-Alkyl,

und wobei

$R^4$ H, nieder-Alkyl, -Alkoxy, -Alkoxymethyl,

$R^5$ nieder-Alkyl, -Halogenalk(en)yl, -Halogenalkinyl,

$R^6$, $R^7$ nieder-Alkyl, Halogen,

A Halogen, nieder-Alkyl, -Alkoxy, -Halogenalkyl, -Halogenalkoxy, CN, $NO_2$,

m 0 bis 4,

n 1 bis 3,

B nieder-Alkyl, -Alkenyl, -Alkinyl, Cycloalkyl,

X H, Halogen, nieder-Halogenalkyl, -Thioalkyl, -Alkoxy, -Alkyl,

bedeuten und Halogen, Fluor, Chlor, Brom oder, im Falle $R^2$ auch Iod sein kann.

In der Formel I steht $R^1$ für einen mindestens dreifach substituierten Cyclopropanrest, insbesondere einen 3-(2,2-Dihalogenvinyl)-2,2-dimethylcyclopropyl, beispielsweise 3-(2,2-Dichlorvinyl)-2,2-dimethylcyclopropyl, den 3-(2,2-Dibromvinyl)-2,2-dimethylcyclopropyl oder 3-(2,2-Difluorvinyl)-2,2-dimethyl-cyclopropyl; 3-(2-Halogenethinyl)-2,2-dimethyl-cyclopropyl, beispielsweise für 3-(2-Chlorethinyl)-2,2-dimethylcyclopropyl oder 3-(2-Bromethinyl)-2,2-dimethylcyclopropyl; 3-(Alkoxy)-2,2-dimethylcyclopropyl, beispielsweise 3-Ethoxy-2,2-dimethylcyclopropyl; 3-Alkoxymethyl-2,2-dimethylcyclopropyl, beispielsweise 3-Methoxymethyl-2,2-dimethylcyclopropyl; Tetraalkylcyclopropyl, insbesondere für Tetramethylcyclopropyl; 3,3-Dihalogen-2,2-dialkylcyclopropyl, insbesondere 3,3-Dichlor-2,2-dimethylcyclopropyl oder den 3,3-Dibrom-2,2-dimethylcyclopropyl; oder 3-(Halogenalkyl)-2,2-dimethylcyclopropyl, wie z. B. 3-(Tetrabromethyl)-2,2-dimethylcyclopropyl oder 3-(2,2-Dichlor-1,2-dibromethyl)-2,2-dimethyl-cyclopropyl oder einem Rest der Formel

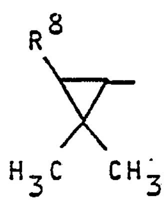

in der $R^8$ für Propen-2-yl, Isobutenyl oder 3-Methylbuten-2-yl steht.

$R^1$ kann weiterhin einen Rest der Formel

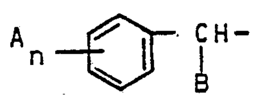

bedeuten, der einen bis drei gleiche oder verschiedene Substituenten A trägt. Substituent A kann in diesem Fall z. B. sein Fluor, Chlor oder Brom ; Halogenmethyl, beispielsweise Trifluormethyl, Difluormethyl, Trichlormethyl ; Halogenmethoxy, beispielsweise Difluormethoxy, Trifluormethoxy ; Cyano, Nitro ; unverzweigtes oder verzweigtes Alkyl- oder Alkoxy mit bis zu 3 Kohlenstoffatomen, beispielsweise Methyl, Methoxy, Ethyl, Ethoxy, n-Propyl, Isopropyl, n-Propoxy, Isopropoxy.

Bevorzugt ist Substituent A, wenn es im Substituenten $R^1$ auftritt, in 4-Stellung angeordnetes Halogen, Halogenalkoxy.

B kann unverzweigtes oder verzweigtes Alkyl, Alkenyl oder Alkinyl mit bis zu 4 Kohlenstoffatomen, beispielsweise Methyl, Ethyl, insbesondere Isopropyl, ferner tert.-Butyl, Isobutyl, Allyl, Isopropenyl, Propargyl oder einen alicyclischen Rest mit z. B. 3 bis 7 Kohlenstoffatomen, beispielsweise Cyclohexyl und insbesondere Cyclopropyl oder einen heterocyclischen Rest mit bis zu 3 Heteroatomen (N oder 0) bedeuten.

$R^2$ bedeutet Wasserstoff, Cyan, unverzweigtes oder verzweigtes Alkyl, Alkenyl, Alkinyl, Halogenalkyl oder Halogenalkenyl mit bis zu 3 Kohlenstoffatomen, beispielsweise Methyl, Ethyl, n-Propyl, Isopropyl, Vinyl, Allyl, n-Propenyl, Isopropenyl, Ethinyl, n-Propinyl, Isopropinyl, Trifluormethyl, Trichlormethyl, Difluorvinyl und ferner Halogen, insbesondere Iod.

$A_m$ bzw. $A_n$ in Formel I bedeuten Wasserstoff oder bis zu vier bzw. bis zu 3 unabhängig voneinander gewählte Substituenten, nämlich Halogen, wie Fluor, Chlor, Brom, unverzweigtes oder verzweigtes Alkyl, Alkoxy, Alkenyl oder Alkinyl mit bis zu 5 (bevorzugt bis zu 3) Kohlenstoffatomen, beispielsweise Methyl, Ethyl, Propyl, Isopropyl, n-Butyl, tert.-Butyl, Isobutyl, Pentyl ; Methoxy, Ethoxy, n-Propoxy, Isopropoxy, Butoxy- oder Pentoxy ; Vinyl, Allyl, n-Propenyl, Isopropenyl, n-Butenyl, n-Pentenyl, 1-Methyl-n-propenyl, 1-Methyl-n-propenyl, 1-Methyl-n-butenyl ; Ethinyl, n-Propinyl, Isopropinyl, n-Butinyl, n-Pentinyl, 1-Methylpropinyl, 1-Methyl-butinyl, sowie CN- und $NO_2$-Gruppen.

$R^3$ steht insbesondere für Wasserstoff, ferner für Halogen, wie Fluor, Chlor, Brom, Methyl-, Ethyl-, Propyl-, Isopropyl.

X bedeutet Wasserstoff, bevorzugt Halogen, wie Fluor, insbesondere Chlor und Brom, und ferner beispielsweise Methyl, Ethyl, Propyl, Isopropyl, Methoxy, Ethoxy, Thiomethyl, Chlormethyl, Brommethyl, Trifluormethyl, Trichlormethyl.

Es versteht sich, daß die Verbindungen der Formel I in jedem Falle in mindestens einem Paar optischer Antipoden, in vielen Fällen auch in Form von Diastereomeren-Vielfachen vorkommen können, die je nach den Ausgangsstoffen und den Umsetzungsbedingungen in einheitlicher Form oder als Gemische auftreten. Die Gemische können in üblicher Weise in ihre sterisch einheitlichen Bestandteile aufgetrennt werden ; ihre biologische Wirkung ist in Einzelfällen von ihrer sterischen Konfiguration abhängig.

Die Ester der Formel I können durch Umsetzung entsprechender Säurehalogenide in Gegenwart eines säurebindenden Mittels mit entsprechenden Halogenvinylbenzylalkoholen erhalten werden.

Natürlich kann man die Ester auch aus entsprechenden Carbonsäuren mit entsprechenden Halogenvinylbenzylhalogeniden in Gegenwart eines säurebindenden Mittels herzustellen.

Schließlich kann man die erfindungsgemäßen Ester auch aus den freien Carbonsäuren und Alkoholen unter Veresterungsbedingungen oder aus vorhandenen Estern mit entsprechenden Carbonsäuren bzw. Alkoholen unter Umesterungsbedingungen erhalten.

Die benötigten Carbonsäurehalogenide bzw. freien Carbonsäuren bzw. Carbonsäuren sind mindestens teilsweise aus der GB-PS 1 446 304 und der US-PS 3 981 903 bekannt. Soweit sie im Einzelfalle neu sind, können sie mit den benötigten und erfindungsgemäß wesentlichen Halogenvinylbenzylverbindungen auf an sich bekannte Weise hergestellt werden (vgl. DE-OS 26 33 551 und DE-OS 32 39 288), beispielsweise zur Einführung des Halogenvinylrestes in den Benzylrest durch Umsetzung eines Aldehyds mit einem entsprechenden Wittig-Reagens oder durch Hydroborierung, bzw. Hydroaluminium oder Halogenwasserstoffaddition von Alkinen (Houben-Weyl, Methoden der organischen Chemie, 4. Auflage, Bd. V/1 b, S. 795 ff). Die zur Herstellung der Wirkstoffe benötigten Zwischenprodukte sind z. T. bekannt, z. T. neu ; sie können in jedem Fall nach bekannten Verfahren hergestellt werden. Hingewiesen wird in diesem Zusammenhang z. B. auf US-PS 3 979 519, US-PS 3 981 903, BE-PS 801 946, DE-OS 2 365 555, DE-OS 2 231 312).

Die benötigten Benzylhalogenide, Alkohole bzw. Aldehyde für die erfindungsgemäßen Ester der Formel I sind mindestens zum Teil neu.

Sie können nach bekannten Verfahren, wie z. B. Halogenierung mit N-Bromsuccinimid, Reduktion von Säuren oder Estern bzw. Oxidation von Alkoholen aus den z. T. bekannten entsprechenden Halogenaromaten, Toluolen bzw. Benzoesäuren hergestellt werden.

Die Herstellung dieser Halogenaromate, Toluole bzw. Benzoesäuren sind z. B. beschrieben in :

GB 2 078 238 ; JA 4 701 900-Q ; US 3 336 335 ; EP 53 687 ; EP 35 724 ; Z. org. Chim. 1965, 1998-2002 ; Liebigs Ann. Chem. 1980, 2061-2071 ; J. Org. Chem. 36, 1438-1440 (1971) ; J. Am. Chem. Soc. 1983, 2388-93.

Herstellungsbeispiele für die erfindungsgemäßen Benzylhalogenide, Alkohole und Aldehyde

A) 2-(2,2-Dichlorvinyl)-benzylbromid

211 g (1,13 mol) 2-(2,2-Dichlorvinyl)-toluol, 251 g (1,41 mol) N-Bromsuccinimid und 2 g Azo-isobutyronitril werden in 1 300 ml trockenem 1,1,1-Trichlorethan unter Rückfluß erhitzt. Nach 1 h gibt man zum Reaktionsgemisch zusätzlich 1 g AIBN und erhitzt eine weitere Stunde.

Man kühlt ab, filtriert über Kieselgel, engt die Lösung ein und fraktioniert bei 0,05 mm Hg.

Man erhält eine helle Flüssigkeit bei 95 bis 97 °C mit einem Brechungsindex $n_D^{23}$ 1.6070 in einer Ausbeute von 258,6 g.

| | | | | |
|---|---|---|---|---|
| Theorie : | C 40,64 | H 2,65 | Cl 26,66 | Br 30,04 |
| Gef. : | C 40,1 | H 2,9 | Cl 26,5 | Br 30,5 |

B) 2-(2,2-Dichlorvinyl) benzylacetat

235 g (0,88 mol) 2-(2,2-Dichlorvinyl) benzylbromid, 144 g (1,76 mol) Na-Acetat werden in 220 ml Eisessig und 300 ml Dimethylformamid suspendiert und mit 2 g KJ 5 h bei 140 °C gerührt.

Man läßt abkühlen und versetzt den Ansatz unter Rühren mit $H_2O$ und Ether. Der Ether wird abgetrennt und die wäßrige Phase noch einmal mit Ether ausgeschüttelt. Die vereinigten Etherphasen werden mit $NaHCO_3$-Lösung und Wasser gewaschen, getrocknet und eingeengt. Das ölige Produkt wird mit Toluol aufgenommen und über Kieselgel filtriert.

Man erhält nach Einengen der Toluollösung ein hellgelbes Öl mit dem Brechungsindex $n_D^{23}$ 1.5484 in einer Ausbeute von 135 g.

| | | | | |
|---|---|---|---|---|
| Theorie : | C 53,60 | H 4,11 | O 13,06 | Cl 28,93 |
| Gef. : | C 53,6 | H 4,3 | O 13,3 | Cl 28,7 |

C) 2-(2,2-Dichlorvinyl) benzylalkohol

133 g (0,54 mol) 2-(2,2-Dichlorvinyl) benzylacetat werden in 200 ml Ethanol gelöst und zu 750 ml einer kalt gesättigten ethanolischen KOH-Lösung bei Raumtemperatur gegeben. Nach 2 h wird der Ansatz eingeengt, mit $H_2O$ aufgenommen und mehrmals mit Ether ausgeschüttelt. Die vereinigten Etherphasen werden mit $H_2O$ gewaschen, getrocknet und eingeengt.

Man erhält ein hellgelbes Öl mit dem Brechungsindex $n_D^{21}$ 1.5891 in einer Ausbeute von 105 g.

| | | | | |
|---|---|---|---|---|
| Theorie : | C 53,23 | H 3,97 | O 7,88 | Cl 34,92 |
| Gef. : | C 53,0 | H 4,0 | O 7,6 | Cl 35,1 |

D) 2-(2,2-Dichlorvinyl) benzaldehyd

20,3 (0,1 mol) 2-(2,2-Dichlorvinyl) benzylalkohol werden zu einer Mischung von 15,7 g (0,16 mol) $H_2SO_4$ conc und 19,8 g (1,1 mol) $H_2O$ gegeben. Man erwärmt auf 60 °C und tropft eine Lösung von 11,9 g (0,04 mol) $Na_2Cr_2O_7$ in 8 ml $H_2O$ so dazu, daß die Temperatur auf 100 °C steigt. Man rührt 1/2 h bei dieser Temperatur nach, kühlt ab, versetzt mit 20 ml $H_2O$ und schüttelt mehrmals mit Toluol aus. Die Toluol-Lösungen werden vereinigt, über Kieselgel filtriert und eingeengt.

Man erhält ein helles Öl mit dem Brechungsindex $n_D^{22}$ 1.5983 in einer Ausbeute von 13,1 g.

| | | | | |
|---|---|---|---|---|
| Theorie : | C 53,77 | H 3,01 | O 7,96 | Cl 35,27 |
| Gef. : | C 53,5 | H 3,3 | O 7,7 | Cl 35,4 |

E) 2-(2,2-Dichlorvinyl)-($\alpha$-isopropyl)-benzylalkohol

3,6 g (0,15 mol) Mg-Späne werden mit 10 ml abs. Ether überschichtet. Dazu tropft man 18,5 g (0,15 mol) 2-Brompropan in 50 ml absol. Ether so zu, daß die Mischung gelinde siedet. Man läßt bei dieser Temperatur 1 h nachrühren und kühlt auf Raumtemperatur ab. Danach tropft man langsam 15 g (0,075 mol) 2-(2,2-Dichlorvinyl)-benzaldehyd in 50 ml Ether zu. Man rührt 1 h bei 40 °C nach, kühlt auf 0 °C ab und versetzt langsam mit 150 ml $NH_4Cl$-Lösung. Man trennt die Ether-Phase ab, wäscht mit $NaHCO_3$-Lösung und Wasser, trocknet und engt ein.

Das zurückbleibende Öl wird säulenchromatographisch über Kieselgel mit Toluol/Aceton = 9/1 als Elutionsmittel aufgetrennt. Man erhält 12 g eines hellgelben Öles.

| | | | | |
|---|---|---|---|---|
| Theorie : | C 58,31 | H 6,52 | O 6,47 | Cl 28,69 |
| Gef. : | C 58,6 | H 6,2 | O 6,9 | Cl 28,5 |

Herstellbeispiel 1

3-(2,2-Dichlorvinyl)-2,2-dimethyl-cyclopropancarbonsäure-(3-(2,2-dibromvinyl-($\alpha$-cyan) benzyl)-ester

In einem zweiphasigen Reaktionsgemisch werden bei Raumtemperatur 6,1 g (0,021 mol) 3-(2,2-Dibromvinyl)-benzaldehyd in 50 ml Ether, 1,5 g (0,023 mol) KCN in 20 ml Wasser, 5,0 g (0,022 mol) 3-(2,2-Dichlorvinyl)-2,2-dimethyl-cyclopropancarbonsäurechlorid und 0,5 g Triethylbenzylammoniumchlorid verrührt und die Durchmischung über Nacht bei 25 °C fortgesetzt. Man trennt und schüttelt 2x mit Ether aus. Die etherische Lösung wird mit Natriumhydrogensulfit, $NaHCO_3$ und Wasser gewaschen, getrocknet über $Na_2SO_4$ und eingeengt.

Man gewinnt 9,1 g (Ausbeute 91 %) eines gelblichen Öls, das über eine Kieselgel-Säule mit Toluol als Elutionsmittel gereinigt wird.

| | | | | | | |
|---|---|---|---|---|---|---|
| Ber. : | C 42,56 | H 2,98 | O 6,30 | N 2,76 | Cl 13,96 | Br 31,46 |
| Gef. : | C 42,6 | H 3,0 | O 5,9 | N 2,7 | Cl 13,5 | Br 31,6 |

Herstellbeispiel 2

2-(4-Chlorphenyl)-isovaleriansäure-(2-(2,2-dichlorvinyl)-benzyl)-ester

2,8 g (0,012 mol) 2-(4-Chlorphenyl)-isovaleriansäurechlorid in 50 ml absol. Toluol werden zu 1,2 g (0,013 mol) τ-Picolin und 3,2 g (0,015 mol) 2-(2,2-Dichlorvinyl)-benzaldehyd bei Raumtemperatur unter Rühren in einen Erlenmeyerkolben eingetragen. Nach 30 Minuten wird über Kieselgel abgenutscht und mit Toluol nachgewaschen.

Nach Einengen gewinnt man 3,9 g (82 % Ausbeute) eines Öles mit dem Brechungsindex $n_D^{21}$ : 1,5620.

| | | | | |
|---|---|---|---|---|
| Ber. : | C 60,40 | H 4,82 | O 8,05 | Cl 26,74 |
| Gef. : | C 59,9 | H 4,8 | O 7,7 | Cl 26,4 |

Die in der nachstehenden Tabelle mit physikalischen Kennzeichen versehenen Verbindungen wurden unter entsprechender Abwandlung der vorstehenden Herstellbeispiele erhalten.

Die nicht näher charakterisierten Verbindungen lassen sich auf die gleiche Weise aus entsprechenden Vorprodukten erhalten und lassen aufgrund struktureller Ähnlichkeit zu den näher untersuchten Verbindungen eine gleichartige Wirkung erwarten.

Tabelle 1

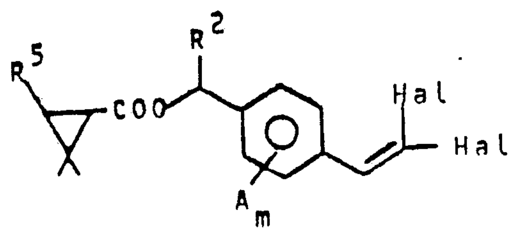

| Nr. | $R^5$ | $R^2$ | Hal | $A_m$ | $n_D$ [°C] |
|---|---|---|---|---|---|
| 3 | $Cl_2C=CH$ | H | Br | H | 1,5912 [22] |
| 4 | $Cl_2C=CH$ | CN | Br | H | 1,6070 [23] |
| 5 | $Cl_2C=CH$ | Vinyl | Br | H | 1,5827 [23] |
| 6 | $Cl_2C=CH$ | H | Cl | 2-F | 1,5628 [22] |
| 7 | $Cl_2C=CH$ | H | Cl | 2-Cl, 6-Cl | 1,5765 [18] |
| 8 | $Cl_2C=CH$ | H | Cl | 2 Cl | 1,5798 [22] |
| 9 | $Cl_2C=CH$ | CN | Cl | 2 Cl | (zähes Öl) |
| 10 | $Cl_2C=CH$ | CN | Cl | H | 1,5740 [23] |
| 11 | $Cl_2C=CH$ | H | Cl | H | 1,5764 [24] |
| 12 | $Cl_2C=CH$ | Vinyl | Cl | H | 1,5729 [21] |
| 13 | $Cl_2C=CH$ | i-Propyl | Cl | H | 1,5446 [21] |
| 14 | $Cl_2C=CH$ | Ethinyl | Cl | H | |

Tabelle 1 (Fortsetzung)

| Nr. | $R^5$ | $R^2$ | Hal | $A_m$ | $n_D$ [°C] |
|---|---|---|---|---|---|
| 15 | $Cl_2C{=}CH$ | Ethinyl | Cl | 2-Cl, 6-Cl | |
| 16 | $Cl_2C{=}CH$ | Ethinyl | Cl | 2-F | |
| 24 | $ClC{\equiv}C$ | H | Br | 2-F, 6-F | |
| 25 | $BrC{\equiv}C$ | CN | Br | 2-Cl, 6-$OCH_3$ | |
| 26 | $BrC{\equiv}C$ | Ethinyl | Cl | H | |
| 27 | $BrC{\equiv}C$ | CN | Cl | H | |
| 28 | $Br_2C{=}CH$ | Vinyl | Cl | H | 1,5805 [21] |
| 29 | $Br_2C{=}CH$ | i-Propyl | Cl | H | 1,5606 [21] |
| 30 | $Br_2C{=}CH$ | H | Cl | 2-Cl | 1,5981 [21] |
| 31 | $Br_2C{=}CH$ | H | Cl | 2-Cl, 6-Cl | 1,5970 [20] |
| 32 | $Br_2C{=}CH$ | H | Cl | 2-F | 1,5820 [23] |
| 33 | $Br_2C{=}CH$ | H | Br | H | 1,6133 [23] |
| 34 | $Br_2C{=}CH$ | Ethinyl | Cl | H | |
| 35 | $Br_3C{-}CHBr$ | Ethinyl | Cl | 2-F, 6-F | |
| 36 | $Cl_2BrC{-}CHBr$ | CN | Br | 2-F, 6-Cl | |
| 37 | H | CN | Cl | H | |

Tabelle 2

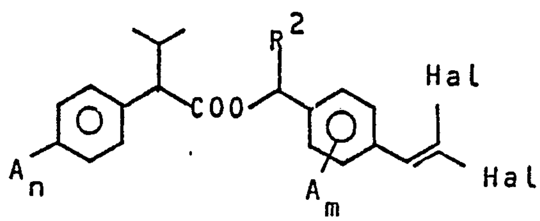

| Nr. | $A_n$ | $R^2$ | Hal | $A_m$ | $n_D$ [°C] |
|---|---|---|---|---|---|
| 38 | Cl | H | Br | H | 1,5938 [23] |
| 39 | Cl | H | Cl | 2-F | 1,5660 [22] |
| 40 | Cl | H | Cl | 2-Cl | 1,5796 [22] |
| 41 | Cl | H | Cl | H | 1,5790 [24] |
| 42 | Cl | CN | Cl | H | 1,5739 [23] |
| 43 | Cl | Vinyl | Cl | H | 1,5632 [21] |
| 44 | Cl | i-Propyl | Cl | H | 1,5500 [21] |
| 45 | Cl | Ethinyl | Cl | H | |
| 46 | $OCH_3$ | H | Cl | 2-Br | |
| 47 | $OC_2H_5$ | CN | Br | 2-F, 6-F | |
| 48 | $OCF_3$ | H | F | 2-Cl, 3-Br | |
| 49 | $OCHF_2$ | Ethinyl | Cl | 2-F, 6-Cl | |
| 50 | $CH_3$ | H | F | H | |
| 51 | $CHF_2$ | CN | Cl | 2-$CF_3$ | |

Tabelle 3

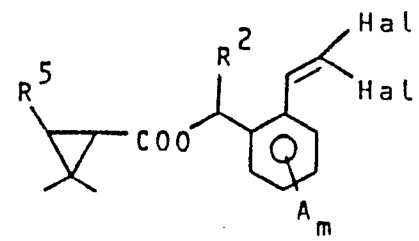

| Nr. | $R^5$ | $R^2$ | Hal | $A_m$ | $n_D$ [°C] |
|---|---|---|---|---|---|
| 52 | $Cl_2C=CH$ | H | Br | H | 1,5797 [23] |
| 53 | $Cl_2C=CH$ | CN | Br | H | 1,5760 [23] |
| 54 | $Cl_2C=CH$ | H | Cl | H | 1,5620 [21] |
| 55 | $Cl_2C=CH$ | CN | Cl | H | 1,5575 [21] |
| 56 | $Cl_2C=CH$ | i-Propyl | Cl | H | 1,5398 [21] |
| 57 | $Cl_2C=CH$ | Ethinyl | Cl | H | |
| 58 | $Cl_2C=CH$ | H | Cl | 4-$CF_3$ | |
| 59 | $Cl_2C=CH$ | Vinyl | Cl | 4-$CH_3$, 6-F | |
| 60 | $Cl_2C=CH$ | CN | Br | 4-$OCH_3$, 6-Cl | |
| 61 | $Br_2C=CH$ | H | Cl | H | 1,5827 [21] |
| 62 | $Br_2C=CH$ | CN | Cl | H | 1,5768 [21] |

Tabelle 5

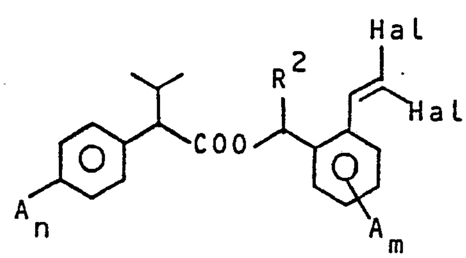

| Nr. | $A_n$ | $R^2$ | Hal | $A_m$ | $n_D$ [°C] |
|---|---|---|---|---|---|
| 79 | Cl | H | Br | H | 1,5810 [23] |
| 80 | Cl | CN | Br | H | 1,5760 [23] |
| 81 | Cl | CN | Cl | H | 1,5588 [21] |
| 82 | Cl | i-Propyl | Cl | H | 1,5579 [21] |
| 83 | Cl | Ethinyl | Cl | H | |
| 84 | $OCF_3$ | H | Cl | 4-Ethinyl | |
| 85 | Cl | CN | Br | 4-Dimethylvinyl | |

Tabelle 6

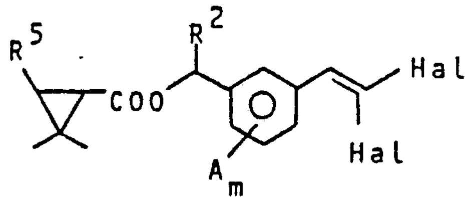

| Nr. | $R^5$ | $R^2$ | Hal | $A_m$ | $n_D$ [°C] |
|---|---|---|---|---|---|
| 86 | $Cl_2C=CH$ | H | Cl | 2-Cl | 1,5789 [22] |
| 87 | $Cl_2C=CH$ | H | Cl | H | 1,5728 [24] |
| 88 | $Cl_2C=CH$ | CN | Cl | H | 1,5722 [23] |
| 89 | $Cl_2C=CH$ | Vinyl | Cl | H | 1,5627 [22] |
| 90 | $Cl_2C=CH$ | i-Propyl | Cl | H | 1,5420 [21] |
| 91 | $Cl_2C=CH$ | $CH_3$ | Cl | H | zähes Öl |
| 92 | $Cl_2C=CH$ | H | Br | H | 1,5884 [22] |
| 93 | $Cl_2C=CH$ | Ethinyl | Cl | H | |
| 94 | BrC≡C | CN | Cl | H | |
| 95 | $Br_2C=CH$ | H | Br | H | 1,6090 [23] |
| 96 | $Br_2C=CH$ | i-Propyl | Cl | H | 1,5596 [21] |
| 97 | $Br_2C=CH$ | Ethinyl | Cl | H | |
| 98 | $Br_2C=CH$ | H | Cl | 5-$OCH_3$, 6-Cl | |
| 99 | $Br_2C=CH$ | H | Br | 5 $CF_3$ | |
| 101 | $(CH_3)_2C=CH$ | H | Br | H | 1,5700 [23] |
| 102 | $(CH_3)_2C=CH$ | H | Cl | H | 1,5761 [24] |
| 103 | $(CH_3)_2C=CH$ | CN | Cl | H | 1,5558 [23] |
| 104 | $(CH_3)_2C=CH$ | Vinyl | Cl | H | 1,5462 [22] |
| 105 | $(CH_3)_2C=CH$ | i-Propyl | Cl | H | 1,5278 [21] |

Tabelle 7

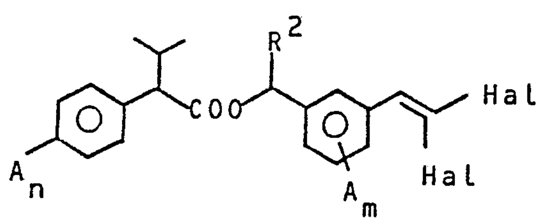

| Nr. | $A_n$ | $R^2$ | Hal | $A_m$ | $n_D$ [°C] |
|---|---|---|---|---|---|
| 106 | Cl | H | Cl | H | 1,5537 [24] |
| 107 | Cl | i-Propyl | Cl | H | 1,5444 [21] |
| 108 | Cl | H | Br | H | 1,5874 [22] |
| 109 | Cl | H | Cl | 2-Cl | 1,5812 [22] |
| 110 | Cl | Ethinyl | Cl | H | |

Die Wirkung einer repräsentativen Auswahl von Stoffen auf Insekten und Spinnentiere ist den nachfolgenden Anwendungsbeispielen zu entnehmen.

Als Vergleichsmittel setzten wir dabei das bekannte, strukturell nahekommende Handelsprodukt Allethrin ein.

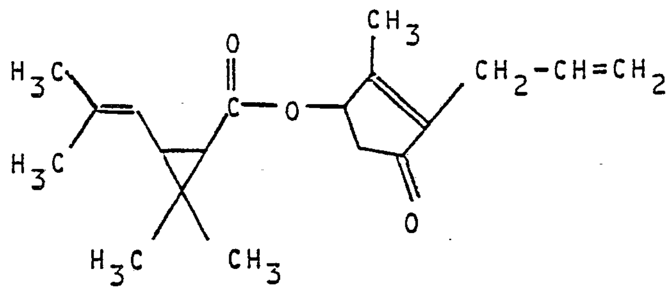

1. Dauerkontakt auf Stubenfliegen (Musca domestica)

Petrischalen mit 10 cm Durchmesser werden innen mit der azetonischen Lösung der Wirkstoffe behandelt.

Nach Verdunsten des Lösungsmittels besetzt man die Schalen mit 20 vier Tage alten Stubenfliegen. Die Mortalität wird nach 4 Stunden ermittelt.

Stubenfliegen

| Beispiel | mg | % Mort. |
|---|---|---|
| 1 | 0,01 | 100 |
| 2 | 2,0 | 100 |
| 3 | 2,0 | 100 |
| 52 | 0,2 | 100 |
| 53 | 0,2 | 80 |
| 54 | 0,2 | 100 |
| 55 | 0,2 | 100 |
| 61 | 0,2 | 100 |
| 62 | 0,2 | 100 |
| 63 | 2,0 | 100 |
| 68 | 0,2 | 80 |
| 76 | 2,0 | 100 |
| 79 | 2,0 | 100 |
| 86 | 0,2 | 100 |
| 87 | 0,2 | 100 |
| 88 | 0,02 | 100 |
| 89 | 0,2 | 100 |
| 92 | 0,01 | 100 |
| 95 | 0,02 | 100 |
| 96 | 2,0 | 100 |
| 101 | 0,2 | 100 |
| 102 | 0,2 | 100 |
| 103 | 0,01 | 80 |
| 104 | 2,0 | 100 |
| 106 | 0,2 | 100 |
| 108 | 0,2 | 100 |

2. Putella maculipennis, Kohlschaben-Raupen ; Fraß- und Kontaktwirkung

Blätter von jungen Kohlpflanzen werden 3 Sekunden in die wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt.

Nach 48 Stunden beurteilt man die Wirkung.

| Beispiel | % | % Mort. |
|---|---|---|
| 10 | 0,01 | 100 |
| 54 | 0,01 | 100 |
| 88 | 0,01 | 100 |
| 92 | 0,004 | 100 |
| Vergleichsmittel | 0,02 | 100 |
| | 0,01 | <70 |

3. Kontaktwirkung auf Moskito-Larven (Aedes aegypti)

200 ml Leitungswasser werden mit der Wirkstoffaufbereitung versetzt und darauf mit 30-40 Moskito-Larven im 4. Larvenstadium besetzt.

Die Versuchstemperatur beträgt 20 °C. Nach 24 Stunden wird die Wirkung ermittelt.

| Beispiel. | ppm | % Mort. |
|---|---|---|
| 1 | 0,01 | 100 |
| 52 | 0,04 | 100 |
| 61 | 0,01 | 100 |
| 66 | 0,04 | 100 |
| 87 | 0,01 | 100 |
| 88 | 0,01 | 100 |
| 89 | 0,04 | 100 |
| 95 | 0,02 | 100 |
| Vergleichsmittel | 0,1 | 100 |
| | 0,04 | <60 |

4. Wirkung auf Spinnmilben (Tetranychus telarius) (Test A)

Getopfte Buschbohnen, die das erste Folgeblattpaar entwickelt haben und einen starken Besatz aller Stadien der Spinnmilbe Tetranychus telarius tragen, werden in der Spritzkabine mit der wäßrigen Wirkstoffaufbereitung tropfnaß gespritzt. Die Pflanzen kommen dazu auf einen Drehteller und werden von allen Seiten mit 50 ml Spritzbrühe besprüht. Der Sprühvorgang dauert ca. 22 Sekunden.

Nach 8 Tagen werden die Pflanzen auf lebende Spinnmilben untersucht.

| Beispiel | % | % Mort. |
|---|---|---|
| 54 | 0,04 | 100 |
| 61 | 0,04 | 100 |
| 63 | 0,02 | 100 |
| 68 | 0,02 | 100 |
| 106 | 0,05 | 100 |
| Vergleichsmittel | 0,1 | < 70 |

Kontaktwirkung auf Stubenfliegen (Musca domestica) Dauerwirkung behandelter Glasplatten

Aufgerauhte Glasplatten von 15 × 15 cm Seitenlänge werden gleichmäßig mit der acetonischen Wirkstofflösung behandelt. Nach dem Verdunsten des Lösungsmittels werden je 10 4-Tage alte

Stubenfliegen unter einer Petrischale (0 10 cm) auf die Glasplatten gesetzt. Die Mortalität nach 4 Stunden gilt als Richtwert im Versuch.

Die Prüfungen werden in Tagesabständen wiederholt, bis die behandelten Platten keine Wirkung mehr zeigen.

Die Versuchstemperatur beträgt 20-22 °C.

Ergebnis

| | | | |
|---|---|---|---|
| Beispiel 1 | 1 mg | 30 Tage | 100 % Mort. |
| Beispiel 95 | 1 mg | 30 Tage | 100 % Mort. |
| Vergleichsmittel | 1 mg | 4 Tage | 50 % Mort. |

Kontaktwirkung auf Zecken (Ornithodorus moubata)

Die Prüfung erfolgt an jungen Zecken, die erst einmal Blut aufgenommen haben. Je 5 Tiere werden in einen TEEFIX-Beutel 5 Sekunden in die wäßrige Wirkstoffaufbereitung getaucht. Der Beutel wird frei aufgehängt. Nach 48 Stunden ermittelt man die Mortalitätsrate.

Die Versuchstemperatur beträgt 25-26 °C.

Ergebnis

| Beispiel | % | % Mort. |
|---|---|---|
| 1 | 0,04 | 100 |
| 52 | 0,1 | 100 |
| 54 | 0,04 | 100 |
| 66 | 0,1 | 100 |
| 79 | 0,1 | 100 |
| 87 | 0,004 | 100 |
| 89 | 0,02 | 100 |
| 92 | 0,04 | 100 |
| 95 | 0,002 | 100 |
| 102 | 0,1 | 100 |
| 103 | 0,1 | 100 |
| 109 | 0,04 | 100 |

**Patentansprüche**

1. Halogenvinylbenzylester der Formel I

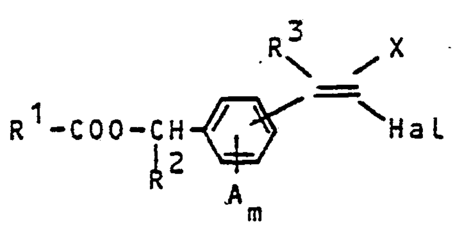

(I)

worin

$R^1$

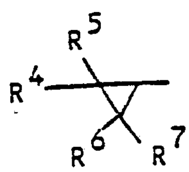 ,

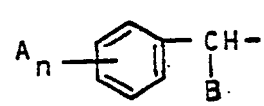 ,

$R^2$ H, CN, nieder-Alkyl, -Alkenyl, -Alkinyl, -Halogenalkyl, -Halogenalkenyl,
$R^3$ H, Halogen, nieder-Alkyl
und wobei
$R^4$ H, nieder-Alkyl, -Alkoxy, -Alkoxymethyl,
$R^5$ nieder-Alkyl, -Halogenalk(en)yl, -Halogenalkinyl,
$R^6$, $R^7$ nieder Alkyl, Halogen,
A Halogen, nieder-Alkyl, -Alkoxy, -Halogenalkyl, -Halogenalkoxy, CN, $NO_2$,
m 0 bis 4,
n 1 bis 3,
B nieder-Alkyl, -Alkenyl, -Alkinyl, Cycloalkyl,
X H, Halogen, nieder-Halogenalkyl, -Thioalkyl, -Alkoxy, -Alkyl,
bedeuten und Halogen, Fluor, Chlor, Brom oder, im Falle $R^2$, auch Iod sein kann.

2. Verfahren zur Herstellung von Estern der Formel I, dadurch gekennzeichnet, daß man ein entsprechendes Säurehalogenid in Gegenwart eines säurebindenden Mittels mit einem entsprechenden Halogenvinylbenzylalkohol umsetzt.

3. Schädlingsbekämpfungsmittel, enthaltend einen Halogenvinylbenzylester der Formel I.

4. Schädlingsbekämpfungsmittel, enthaltend einen festen oder flüssigen Trägerstoff und mindestens einen Ester der Formel I.

5. Verfahren zur Herstellung von Schädlingsbekämpfungsmitteln, dadurch gekennzeichnet, daß man Ester der Formel I mit Streckmitteln und/oder oberflächenaktiven Mitteln mischt.

6. Verfahren zur Bekämpfung von Schädlingen, dadurch gekennzeichnet, daß man Ester der Formel I auf die genannten Schädlinge bzw. deren Lebensraum einwirken läßt.

7. Verwendung von Estern der Formel I zur Bekämpfung von Schädlingen.

## Claims

1. Halovinylbenzyl esters of the formula I

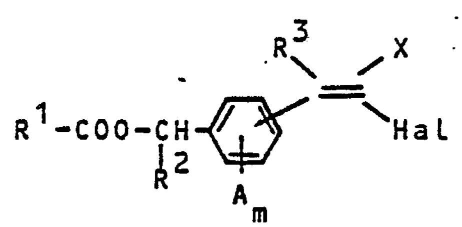

(I)

where
$R^1$ is

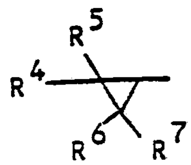 , 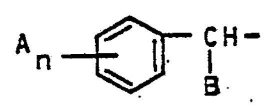 ,

$R^2$ is H, CN, lower alkyl, lower alkenyl, lower alkynyl, lower haloalkyl or lower haloalkenyl,
$R^3$ is H, halogen or lower alkyl,
$R^4$ is H, lower alkyl, lower alkoxy or lower alkoxymethyl,
$R^5$ is lower alkyl, lower haloalk(en)yl or lower haloalkynyl,
$R^6$ and $R^7$ are each lower alkyl or halogen,
A is halogen, lower alkyl, lower alkoxy, lower haloalkyl, lower haloalkoxy, CN or $NO_2$,
m is 0 to 4,
n is 1 to 3,
B is lower alkyl, lower alkenyl, lower alkynyl or cycloalkyl, and
X is H, halogen, lower haloalkyl, lower thioalkyl, lower alkoxy or lower alkyl,
and halogen can be fluorine, chlorine or bromine and, in the case of $R^2$, may furthermore be iodine.

2. A process for the manufacture of esters of the formula I, wherein a corresponding acid halide is reacted, in the presence of an acid-binding agent, with a corresponding halovinylbenzyl alcohol.

3. A pesticidal composition containing a halovinylbenzyl ester of the formula I.

4. A pesticidal composition containing a solid or liquid carrier and at least one ester of the formula I.

5. A process for manufacturing pesticidal compositions, where esters of the formula I are mixed with extenders and/or surfactants.

6. A process for combating pests, wherein esters of the formula I are allowed to act on the pests or their habitat.

7. The use of esters of the formula I for combating pests.

**Revendications**

1. Esters halogénovinylbenzyliques de formule I

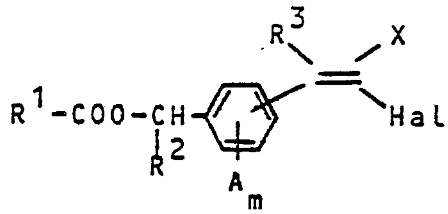

(I)

dans laquelle

$R^1$ représente

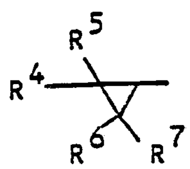

,

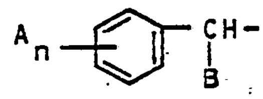

,

$R^2$ représente H, CN, akyle, alcényle, alcynyle, halogéno-alkyle, halogéno-alcényle inférieurs,

$R^3$ H, halogène, alkyle inférieur

$R^4$ H, alkyle, alcoxy, alcoxyméthyle inférieurs,

$R^5$ alkyle, halogéno-alkyle, halogéno-alcényle, halogénoalcynyle inférieurs,

$R^6$, $R^7$ alkyle inférieur, halogène

A halogène, alkyle, alcoxy, halogéno-alkyle, halogéno-alcoxy inférieurs, CN, $NO_2$,

m 0 à 4

n 1 à 3

B alkyle, alcényle, alcynyle inférieurs, cycloalkyle,

X H, halogène, halogéno-alkyle, thioalkyle, alcoxy, alkyle inférieurs,

l'halogène pouvant être fluor, chlore, brome ou, dans le cas de $R^2$, également iode.

2. Procédé de préparation d'esters de formule I, caractérisé par le fait qu'on fait réagir, en présence d'un liant d'acide, un halogénure d'acide correspondant avec un alcool halogénovinylbenzylique correspondant.

3. Moyens de lutte contre les parasites, contenant un ester halogénovinylbenzylique de formule I.

4. Moyens de lutte contre les parasites, contenant un véhicule solide ou liquide et au moins un ester de formule I.

5. Procédé de préparation de moyens de lutte contre les parasites, caractérisé par le fait que l'on mélange des esters de formule I avec des extendeurs et/ou des surfactifs.

6. Procédé de lutte contre les parasites, caractérisé par le fait que l'on fait agir des esters de formule I sur les parasites ou leur biotope.

7. Utilisation d'esters de formule I pour la lutte contre les parasites.